# EUROPEAN PATENT SPECIFICATION

(11) **EP 4 125 680 B1**
(45) Date of publication and mention of the grant of the patent: **28.10.2026**
(21) Application number: 21717170.1
(22) Date of filing: 31.03.2021
(51) Int. Cl.: A61B 34/30, A61B 34/37, B25J 19/00, A61B 18/12

(54) **TESTING UNIT FOR TESTING A SURGICAL ROBOTIC SYSTEM**
PRÜFEINHEIT ZUM PRÜFEN EINES CHIRURGISCHEN ROBOTERSYSTEMS
APPAREIL D'ESSAI POUR TESTER UN SYSTÈME ROBOTIQUE CHIRURGICAL

(30) Priority: 31.03.2020 GB 202004739
(43) Date of publication of application: 08.02.2023
(73) Proprietor: CMR Surgical Limited, Cambridge CB24 9NG (GB)
(72) Inventor: STRAK, Joseph, Cambridge Cambridgeshire CB24 9NG (GB)
(74) Representative: Slingsby Partners LLP
(86) International application number: PCT/GB2021/050796
(87) International publication number: WO 2021/198682

(56) References cited:
- WO-A2-2019/040598
- US-A1- 2011 306 986
- US-A1- 2019 000 578

## Description

### FIELD OF THE INVENTION

This invention relates to a testing unit for testing at least a portion of a surgical robotic system. In particular, the invention relates to a testing unit for coupling to one or more subsystems of a surgical robotic system for testing those subsystems of the surgical robotic system.

### BACKGROUND

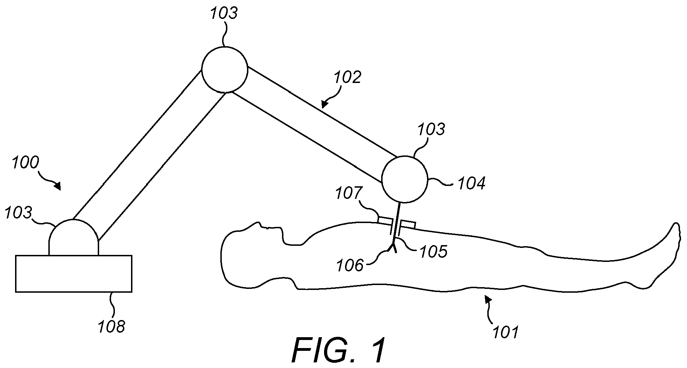

It is known to use robots for assisting and performing surgery. Figure 1 illustrates a typical surgical robot 100 which consists of a base 108 and an arm 102. An instrument 105 is coupled to the arm. The base supports the robot, and is itself attached rigidly to, for example, the operating theatre floor, the operating theatre ceiling or a trolley. The arm extends between the base and the instrument. The arm is articulated by means of multiple flexible joints 103 along its length, which are used to locate the surgical instrument in a desired location relative to the patient. The surgical instrument is attached to the distal end 104 of the robot arm. The surgical instrument penetrates the body of the patient 101 at a port 107 so as to access the surgical site. As illustrated, at its distal end, the instrument comprises an end effector 106 for engaging in a medical procedure. The term 'instrument' encompasses an endoscope for imaging a surgical site, and includes electrosurgical instruments.

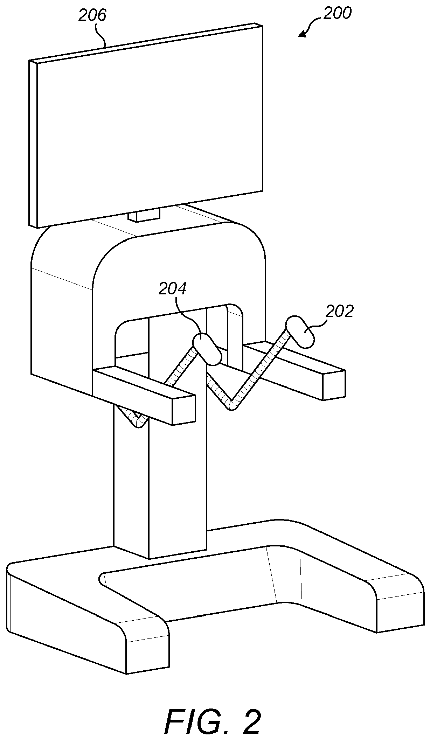

The surgical robot 100 is controlled remotely by an operator (e.g. surgeon) via an operator console 200 shown in Figure 2. The operator console 200 may be located in the same room (e.g. operating theatre) as the surgical robot 100 or remotely from it. The operator console 200 comprises input devices 202, 204 for controlling the state of the arm 102 and/or the instrument 105 attached thereto. The input devices 202, 204 may be handgrips or hand controllers mounted on parallelogram linkages. A control system converts the movement of the hand controllers into control signals to move the arms, joints and/or instrument end effector of a surgical robot. An operator can select when to activate (energise) an electrosurgical tool. The control system can determine that an electrosurgical tool is to be energised and can generate a further control signal accordingly.

The operator console 200 also comprises a display 206. The display 206 is arranged to be visible to a user operating the input devices 202, 204. The display is used to display a video stream of the surgical site (e.g. endoscope video).

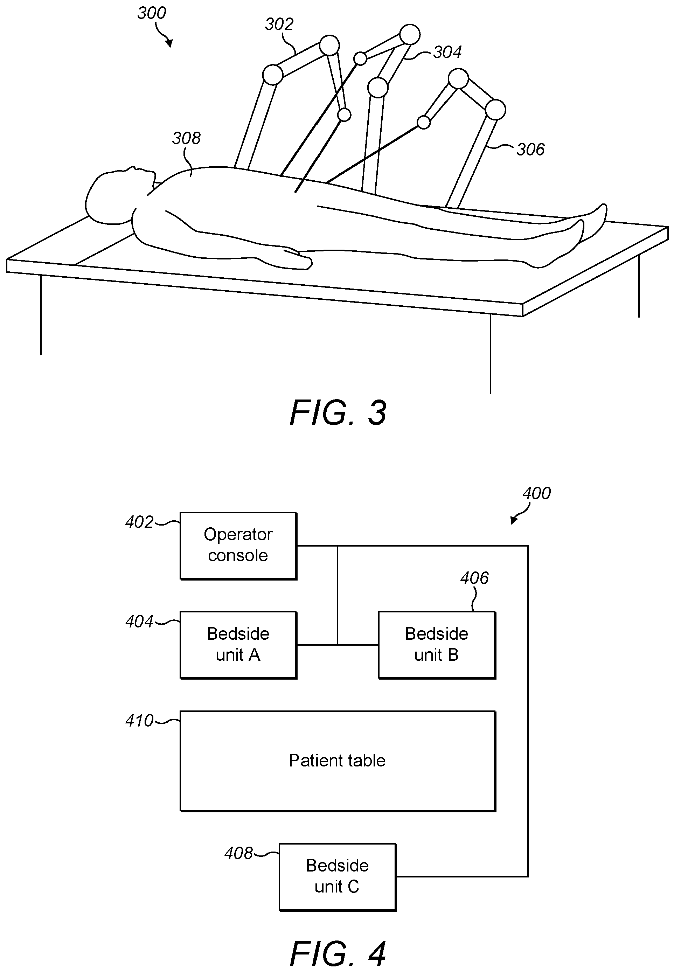

Some surgical procedures may require several surgical robots, each one carrying an instrument or other implement which is used concurrently with the others at the surgical site. Figure 3 illustrates a surgical robotic system 300 with multiple robots 302, 304, 306 operating in a common workspace on a patient 308. For example, surgical robots are often used in endoscopic surgery (e.g. laparoscopic surgery), which also may be referred to as minimally invasive surgery. As is known to those of skill in the art, during an endoscopic procedure the surgeon inserts an endoscope through a small incision or natural opening in the body, such as, but not limited to, the mouth or nostrils. An endoscope is a rigid or flexible tube with a camera attached thereto that transmits real-time images to a video monitor (e.g. display 206) that the surgeon uses to help guide their tools through the same incision/opening or through a different incision/opening. The endoscope allows the surgeon to view the relevant area of the body in detail without having to cut open and expose the relevant area. This technique allows the surgeon to see inside the patient's body and operate through a much smaller incision than would otherwise be required for traditional open surgery. Accordingly, in a typical robotic endoscopic surgery there is an endoscope attached to one surgical robot arm and one or more other surgical instruments, such as a pair of graspers and/or scissors, attached to one or more other surgical robot arms.

The endoscope can interface with the robot arm to which it is mounted, or to another part of the robotic system to provide power and data connections to the endoscope. The power connection can provide power to articulations of the endoscope and/or a light of the endoscope. The data connection can provide control signals for controlling the endoscope. The endoscope can capture still and/or moving images (video). The data connection suitably permits the image(s) to be transferred from the endoscope, for example to the operator console for display and/or storage.

In addition to the endoscope video, data including telemetry data can be passed between the robot arms (and instruments coupled to the robot arms) and the operator console. Such data can comprise control signals for effecting operation of the robot arms and instruments, and sensed data generated by sensors located on the arms and/or instruments. The data can therefore relate to the state of the robotic system. It would be useful to be able to determine a state of the robotic system in a more flexible manner. WO 2019/040598 describes techniques for testing whether an end effector, or component thereof, is correctly or incorrectly installed to a manipulation system. US 2019/000578 relates to emulation of robotic arms and control thereof in a virtual reality environment. US 2011/306986 relates to a surgical robot system using augmented reality or history information and a control method thereof.

### SUMMARY

Various aspects useful for understanding, but not necessarily defining the invention, are set out below. The invention is defined by the independent claims.

According to an aspect of the present invention, there is provided a surgical robotic testing unit for testing a surgical robotic system, the surgical robotic system comprising a first subsystem configured to generate a first signal having a first characteristic behaviour and a second subsystem configured to receive the first signal and to respond to the received first signal, the testing unit being configured to:
emulate the first subsystem by:
   generating an emulated signal representative of the first signal of the first subsystem, the emulated signal having an emulated behaviour that exceeds a boundary of the first characteristic behaviour of the first signal, such that the testing unit is operable to test the second subsystem beyond the capability of the first subsystem;
   transmitting the generated emulated signal for receiving at the second subsystem; and
   receiving a response signal from the second subsystem indicative of the response of the second subsystem to the emulated signal;
analyse the received response signal; and
determine a state of the second subsystem based on the analysis.

The testing unit may be configured to analyse the received response signal by comparing the received response signal with an expected signal. The boundary of the first characteristic behaviour may comprise a maximum signal value, and the testing unit may be configured to generate the emulated signal with a value greater than the maximum signal value.

The boundary of the first characteristic behaviour may comprise a minimum signal value, and the testing unit may be configured to generate the emulated signal with a value less than the minimum signal value.

The testing unit may be configured to generate the emulated signal over a range of values that encompasses a first range of values of the first signal.

The boundary of the first characteristic behaviour may comprise a repeatability indication of the first signal, and the testing unit may be configured to generate the emulated signal with a repeatability greater than the indicated repeatability. The repeatability indication may comprise a standard deviation, and the testing unit may be configured to generate the emulated signal with a standard deviation smaller than that of the repeatability indication.

The boundary of the first characteristic behaviour may comprise a rate of change of values of the first signal, and the testing unit may be configured to generate the emulated signal with a rate of change greater than and/or less than the boundary rate of change.

The testing unit may be configured to generate one or more emulated signals from a set of emulated signals, each emulated signal in the set of emulated signals being representative of a signal that can be generated by the first subsystem.

The testing unit may be configured to:
emulate the second subsystem by:
   generating a further emulated signal representative of a second signal of the second subsystem for receiving at the first subsystem, the second signal having a second characteristic behaviour, and the further emulated signal having an emulated behaviour that exceeds a further boundary of the second characteristic behaviour of the second signal, such that the testing unit is operable to test the first subsystem beyond the capability of the second subsystem;
   transmitting the generated further emulated signal for receiving at the first subsystem; and
   receiving a further response signal from the first subsystem indicative of the response of the first subsystem to the further emulated signal;
analyse the received further response signal with a further expected signal; and
determine a state of the first subsystem based on the comparison.

The testing unit may be configured to analyse the received further response signal by comparing the received further response signal with a further expected signal. The first subsystem may comprise one of a control console and a bedside unit and the second subsystem may comprise the other of the control console and the bedside unit.

The testing unit may be configured to emulate a plurality of control consoles and/or bedside units. The emulated signal may be representative of an arm control signal. The emulated signal may comprise one or more desired joint torques and/or speeds. The emulated signal may be representative of an instrument control signal. The emulated signal may comprise an electrosurgical signal.

The emulated signal comprises a desired range of movement of the arm and/or of the instrument.

According to another aspect of the present invention, there is provided a surgical system comprising:
a surgical robotic system having
   a first subsystem configured to generate a first signal having a first characteristic behaviour, and
   a second subsystem configured to receive the first signal and to respond to the received first signal; and
a surgical robotic testing unit configured to:
   emulate the first subsystem by:
      generating an emulated signal representative of the first signal of the first subsystem, the emulated signal having an emulated behaviour that exceeds a boundary of the first characteristic behaviour of the first signal, such that the testing unit is operable to test the second subsystem beyond the capability of the first subsystem;
      transmitting the generated emulated signal for receiving at the second subsystem; and
      receiving a response signal from the second subsystem indicative of the response of the second subsystem to the emulated signal;
   analyse the received response signal; and
   determine a state of the second subsystem based on the analysis.

According to another aspect of the present invention, there is provided a method of testing a surgical robotic system, the surgical robotic system comprising a first subsystem configured to generate a first signal having a first characteristic behaviour and a second subsystem configured to receive the first signal and to respond to the received first signal, the method comprising:
generating an emulated signal representative of the first signal of the first subsystem, the emulated signal having an emulated behaviour that exceeds a boundary of the first characteristic behaviour of the first signal;
transmitting the generated emulated signal for receiving at the second subsystem;
receiving a response signal from the second subsystem indicative of the response of the second subsystem to the emulated signal;
analysing the received response signal; and
determining a state of the second subsystem based on the analysis, thereby to test the second subsystem beyond the capability of the first subsystem.

According to another aspect of the present invention, there is provided a testing unit configured to perform the method described herein.

According to another aspect of the present invention, there is provided a surgical robotic system comprising a robot having a base and an arm extending from the base to an attachment for an instrument, and a testing unit configured to perform the method described herein.

According to another aspect of the present invention there is provided a non-transitory computer readable storage medium having stored thereon computer readable instructions that, when executed at a computer system, cause the computer system to perform the method as described herein.

### BRIEF DESCRIPTION OF THE DRAWINGS

The present invention will now be described by way of example with reference to the accompanying drawings.

In the drawings:
Figure 1 schematically illustrates an example surgical robot performing an example surgical procedure;
Figure 2 schematically illustrates an example operator console;
Figure 3 schematically illustrates an example surgical robot system with a plurality of surgical robots;
Figure 4 schematically illustrates a typical setup of a surgical robotic system;
Figure 5a schematically illustrates a surgical robotic system with a testing unit;
Figure 5b schematically illustrates another surgical robotic system with a testing unit;
Figure 6 schematically illustrates a coupling arrangement between a testing unit and subsystems of a surgical robotic system;
Figure 7 schematically illustrates an example of an operator console;
Figure 8 schematically illustrates an example of a bedside unit;
Figure 9 schematically illustrates an example of a testing unit; and
Figure 10 shows a method of operating a testing unit.

### DETAILED DESCRIPTION

A surgical robotic system will typically comprise many different subsystems. The subsystems, such as an operator console and one or more bedside units, interact with one another to achieve the functionality of the robotic system as a whole. The operator console can operatively control robot arms and instruments coupled to the bedside units, and can receive responses from the bedside units. It is possible to connect the various subsystems of the robotic system together, and to operate the system, so as to be able to determine the states of the various subsystems or the whole system by monitoring data transferred between the subsystems.

In an improved approach, a surgical robotic testing unit can be coupled to one or more of the subsystems of the robotic system. The testing unit can emulate another subsystem, for example by generating signals and transmitting them to the subsystem under test. The testing unit can monitor the response of the subsystem under test, and can thereby determine a state of the subsystem under test. For example, the testing unit can couple to a bedside unit having an arm mounted thereto. The testing unit can generate drive signals to drive the arm joints in a particular manner. The testing unit can monitor sensor signals, such as from torque and/or acceleration sensors on the arm, to determine how the arm responds to the drive signals. If the arm behaves in an unexpected way, the testing unit can determine that a fault has occurred in the arm. Thus the testing unit can be used to diagnose faults in one or more subsystems. The testing unit can also be used in maintenance testing, by analysing responses to one or more of a series of test signals, which may be predetermined.

An advantage of the testing unit described herein is that it is able to generate signals beyond the capability of the subsystem that it emulates. For instance, it can generate signals outside a range of signals of the subsystem being emulated. The testing unit can suitably generate signals that change at a faster or slower rate than that possible from the subsystem being emulated. The testing unit can suitably generate signals with a higher repeatability than is possible from the subsystem being emulated. This can enable a higher accuracy in testing subsystems.

Enabling the testing unit to test the respective subsystem with a signal having a characteristic that exceeds a boundary signal characteristic of a different subsystem signal means that the testing unit can 'stress-test' the respective subsystem. That is, the testing unit can test the respective subsystem under conditions that are more extreme than would be experienced during standard operation of the surgical robotic system. This testing approach permits a greater confidence in the response of the subsystem during standard operation.

The surgical robotic testing unit is for testing a surgical robotic system. Suitably, the surgical robotic system comprises a first subsystem. The first subsystem can be an operator console or a bedside unit. The first subsystem is configured to generate a first signal having a first characteristic behaviour. The robotic system comprises a second subsystem, such as a bedside unit or an operator console, configured to receive the first signal and to respond to the received first signal. The testing unit is configured to emulate the first subsystem. The testing unit suitably emulates the first subsystem by generating an emulated signal. The emulated signal is representative of the first signal of the first subsystem. The emulated signal has an emulated behaviour. The emulated behaviour exceeds a boundary of the first characteristic behaviour of the first signal, such that the testing unit is operable to test the second subsystem beyond the capability of the first subsystem.

The testing unit can further emulate the first subsystem by transmitting the generated emulated signal for receiving at the second subsystem. The testing unit is further configured to receive a response signal from the second subsystem indicative of the response of the second subsystem to the emulated signal.

The testing unit is configured to compare the received response signal with an expected signal and to determine a state of the second subsystem based on the comparison.

Thus, the testing unit can perform a maintenance or diagnostic test on the second subsystem, by transmitting a desired signal to the second subsystem and monitoring the response of the second subsystem to the transmitted signal.

Generally, the operator console can be considered to be a subsystem of the surgical robotic system. A bedside unit can be considered to be a further subsystem of the surgical robotic system. The subsystems may be taken at a higher level of generality than this. For example, a plurality of bedside units may be coupled together and considered a subsystem as a whole. The subsystems may be taken at a lower level of generality. For example, an instrument or endoscope coupled to an arm can be considered to be a subsystem. A robot arm coupled to a bedside unit can be considered to be a subsystem. A processing or control module within the operator console can be considered to be a subsystem. However, the precise division of what is to be considered a subsystem of the surgical robotic system is not critical.

The present techniques are applicable where a portion of the surgical robotic system can receive an input and generate an output in response to the received input. This enables that portion to be 'queried' by the testing unit. The response of that portion to the testing unit query can be used in regular maintenance and/or fault diagnostics procedures.

In the following discussion, a bedside unit will be considered as a subsystem of the surgical robotic system. The bedside unit may have a robot arm attached thereto. When attached to the bedside unit, the robot arm can also be considered, for the purposes of the present discussion, to be part of that subsystem. The robot arm may have an instrument or endoscope attached thereto. When attached to the arm, the instrument or endoscope can also be considered, for the purposes of the present discussion, to be part of that subsystem. It will be appreciated that the robot arm and instrument need not be part of the bedside unit subsystem.

A typical setup of a surgical robotic system 400 is illustrated in figure 4. Figure 4 shows an operator console 402 coupled to three bedside units: bedside unit A 404, bedside unit B 406 and bedside unit C 408. In the illustrated arrangement, bedside unit C 408 is located to one side of a patient table 410 and bedside units A 404 and B 406 are located to the other side of the patient table 410. Each bedside unit is directly coupled to the operator console 402. In other arrangements, the bedside units can be coupled together in series and the operator console coupled to the first of the bedside units.

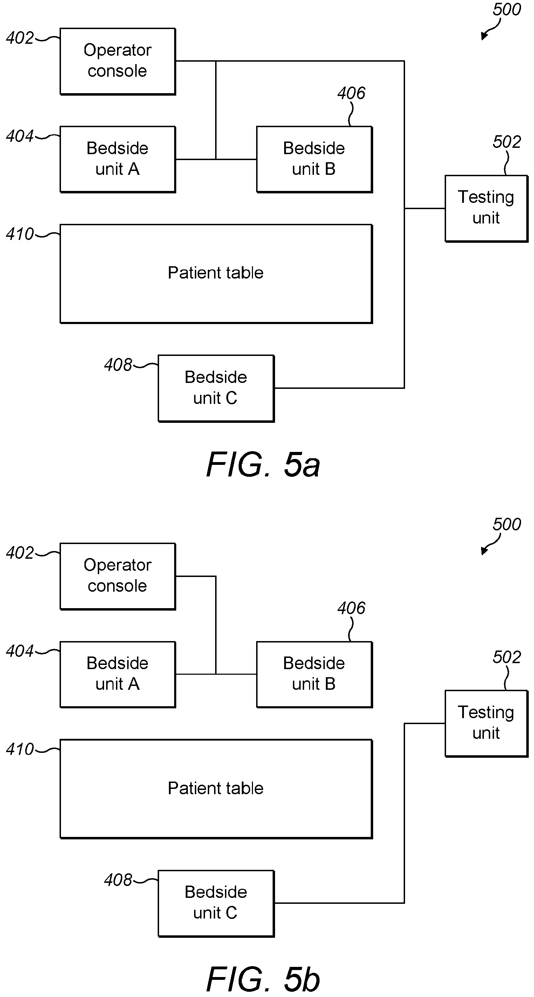

Figure 5a shows a surgical robotic system 500 as in figure 4, but with the addition of a testing unit 502. The testing unit can be provided so as to couple to the operator console and/or any one or more of the bedside units directly. In the example arrangement of figure 5a, the testing unit 502 couples to the bedside units whilst they remain coupled to the operator console. In this arrangement, the testing unit 502 can be operable to test one or more of the operator console and the bedside units without needing the console or bedside units to be disconnected from the other parts of the system. Such an approach enables the testing unit to be coupled to the robotic system in a convenient manner, without necessitating a reorganisation of the robotic system itself.

Thus, the testing unit 502 can be operatively coupled to the robotic system 500 in a quick manner. Where testing is carried out when a surgical procedure is not being performed, being able to couple the testing unit 502 to the robotic system with minimal disruption can reduce the down time for which the robotic system is unable to perform a surgical procedure. Hence a maintenance check or fault diagnostic procedure can be carried out in an efficient manner. If a replacement is needed, for example because there is a fault with an instrument coupled to the robotic system, this can be done before the system is used for another procedure. This can potentially reduce a harmful interruption if the fault is detected, and a replacement carried out, only once another procedure has started. Where no replacement is needed, the testing unit 502 can be decoupled from the surgical system, enabling the surgical system to be ready for another procedure without needing to be connected back together.

Another approach is to couple the testing unit 502 to a subsystem of the surgical robotic system when that subsystem is decoupled from the remainder of the surgical robotic system. This approach is illustrated in figure 5b, which shows the testing unit 502 coupled to bedside unit C 408. Bedside unit C is not coupled to the operator console 402. Such an approach enables the testing unit 502 to test bedside unit C 408 without potential interference from signals sent from the operator console 402. Thus, this alternative approach can enable the operator console to continue communicating with the other bedside units 404, 406, for example for repositioning the arms and/or instruments of those bedside units. Thus, the remaining portions of the surgical robotic system can be prepared for another surgical procedure as testing is being carried out on another portion of the system. This arrangement also permits the operator console 402 to operatively control bedside unit A 404 and bedside unit B 406 during a surgical procedure whilst bedside unit C 408 undergoes testing by the testing unit 502. In this arrangement, bedside unit c 408 need not remain or be located near the patient table 410.

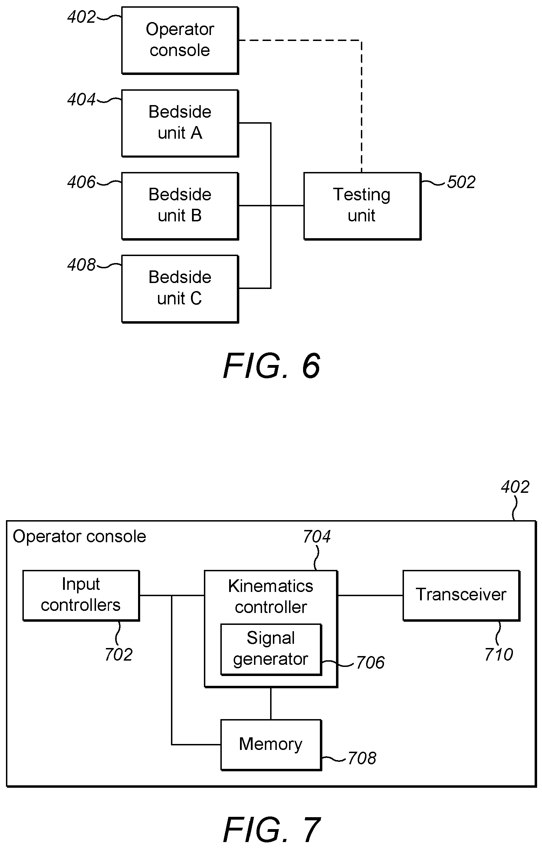

Suitably, the testing unit 502 is configured to couple to up to three bedside units at once, as illustrated in figure 6, though in other configurations the testing unit 502 may couple to more or fewer bedside units. The testing unit 502 is coupled to bedside unit A 404, bedside unit B 406 and bedside unit C 408. In this configuration, the testing unit is suitably configured to emulate the operator console when interfacing with the bedside units. The testing unit can therefore generate appropriate signals such as drive signals for driving arms and/or instruments of the bedside units.

The testing unit 502 is suitably also able to couple to the operator console 402 (as indicated by the dashed line in figure 6). This connection may be at the same time as the connection between the testing unit 502 and the bedside units, but it need not be. In one approach, the testing unit 502 is configured to couple to the operator console 402 when the testing unit 502 is not coupled to a bedside unit. In the configuration where the testing unit 502 is coupled to the operator console 402 (whether or not coupled also to one or more bedside units) the testing unit is suitably configured to emulate one or more bedside units (optionally including respective arms and potentially also surgical instruments and/or an endoscope). In this way, the testing unit can interact with the operator console such that it appears to the operator console as if it is coupled to one or more actual bedside units. The behaviour of the operator console in response to signals emulating those of a bedside unit can thereby be tested by the testing unit 502.

The operator console, bedside unit and testing unit will now be further described with reference to figures 7 to 9. An illustrative example of an operator console is shown in figure 7. The operator console 402 comprises one or more input controllers 702, a kinematics controller 704 having a signal generator 706, a memory 708 and a transceiver 710. Typically two input controllers are provided, for manipulation by the hands of an operator such as a surgeon. Other examples of input controller include a foot pedal, an eye tracking sensor, a gesture sensor, a touch-sensitive interface, an audio sensor such as a voice-activated interface, and so on. The input controllers 702 couple to the kinematics controller 704. The kinematics controller is for processing the signals received from the input controllers and generating appropriate signals such as drive signals for driving the robotic arms and instruments. The kinematics controller 704 is suitably responsive to sensor signals from sensors at the bedside units (for example on the arms and/or instruments). The kinematics controller may comprise the signal generator unit 706 for generating signals for transmission to the bedside units. In some implementations the signal generator need not be provided as a distinct module. In some implementations, the signal generator may be provided as a separate module. The kinematics controller couples to the memory 708, for example to access stored sensor readings relating to the bedside units and/or previously generated signals such as drive signals, from which current drive signals are to be generated. The input controllers 702 may also couple directly to the memory 708, for example to store input controller signals. The kinematics controller couples to the transceiver 710, which facilitates transfer of signals generated by the operator console to another subsystem. The transceiver also facilitates reception of signals from such other subsystems. The received signals may be processed at the operator console, for example at the kinematics controller.

It will be appreciated that the above description of the operator console has focussed on selected elements of an operator console, for the purposes of understanding the present techniques. This description is not necessarily to be taken as a complete description of a practical implementation of such an operator console.

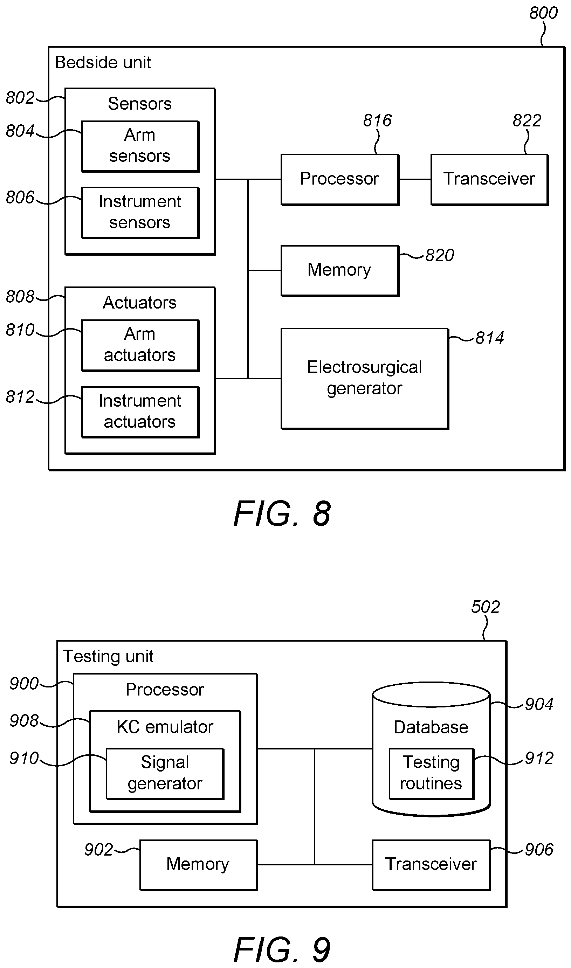

An example of a bedside unit 800 is illustrated in figure 8. The bedside unit comprises sensors 802, including arm sensors 804 and instrument (and/or endoscope) sensors 806. The bedside unit also comprises actuators 808, including arm actuators 810 and instrument (and/or endoscope) actuators 812. The bedside unit, as illustrated also comprises an electrosurgical generator 814. It will be understood that the electrosurgical generator need only be provided where an electrosurgical instrument is coupled to a robot arm. The bedside unit further comprises a processor 816, a memory 820 and a transceiver 822.

The sensors 802 shown schematically in figure 8 will typically be provided on or as part of a robot arm or instrument. The arm sensors 804 are provided on or as part of the surgical robotic arm. The instrument sensors 806 are provided on or as part of the surgical instrument. The instrument sensors may be replaced with endoscope sensors where an endoscope is provided in place of a surgical instrument. In other implementations, the instrument sensors may be located on or as part of the surgical robot arm. Where multiple sensors such as instrument sensors 806 are provided, a subset of the multiple sensors may be provided on or as part of the surgical robot arm and a different subset of the multiple sensors may be provided on or as part of the surgical instrument.

The electrosurgical generator 814 is configured to provide power to an electrosurgical instrument such as a cauteriser. A particular power profile can be supplied by the electrosurgical generator 814 in response to control signals sent by the processor 816. The power provided by the electrosurgical generator can be coupled to the electrosurgical instrument (not shown).

The processor 816 is configured to receive sensor inputs from the sensors 802 and to provide drive signals to the actuators 808. The processor is configured to process the signals in accordance with a control system to effect appropriate control over portions of the bedside unit 800. The processor may operate in response to signals received from another subsystem such as the operator console. The processor may transmit signals to another subsystem such as the operator console.

Suitably one or more of the sensors 802, the actuators 808, the electrosurgical generator 814 and the processor 816 are coupled to the memory 820. This enables signals and/or other data to be saved to the memory and read from the memory.

The processor 816 couples to the transceiver 822, which facilitates transfer of signals generated by the bedside unit 800 to another subsystem. The transceiver also facilitates reception of signals from such other subsystems. The received signals may be processed at the bedside unit, for example at the processor 816.

It will be appreciated that the above description of the bedside unit 800 has focussed on selected elements of a bedside unit (where the robot arm and instrument coupled to the robot arm are considered part of the bedside unit), for the purposes of understanding the present techniques. This description is not necessarily to be taken as a complete description of a practical implementation of such a bedside unit.

An example of a testing unit 502 is illustrated in figure 9. The testing unit comprises a processor 900, a memory 902, a database 904 and a transceiver 906. The processor comprises a kinematics controller emulator 908 for emulating the kinematics controller 704 of the operator console 402. The kinematic controller emulator may comprise a signal generator module 910 for emulating the signal generator 706 of the operator console. The kinematics controller emulator 908 enables the testing unit 502 to emulate the behaviour of the operator console in generating signals such as drive signals for transmission to bedside units. The testing unit 502 is thus able to emulate the operator console, for example when coupled to one or more bedside units.

The processor 900 is also suitably configured to process a signal received from an operator console and to generate appropriate signals in response, such as response signals characteristic of a response of a bedside unit (optionally including emulated sensor signals and/or actuator signals, and/or electrosurgical signals). The testing unit 502 is thus able to emulate one or more bedside units, for example when coupled to an operator console.

The processor 900 is coupled to the memory 902, the database 904 and the transceiver 906. The memory 902 permits the processor to store signals and data relating to the subsystem being emulated by the testing unit 502. The processor is able to access data stored in the memory 902. The database 904 comprises testing routines 912. The testing routines suitably relate to predetermined ways in which the testing unit is to test subsystems of the surgical robotic system, as is described in more detail elsewhere herein.

The transceiver 906 facilitates transfer of signals generated by the testing unit 502 to a subsystem under test. The transceiver 906 also facilitates reception of signals from such a subsystem. The received signals may be processed at the testing unit, for example at the processor 900.

It will be appreciated that the above description of the testing unit 502 has focussed on selected elements of a testing unit for the purposes of understanding the present techniques. This description is not necessarily to be taken as a complete description of a practical implementation of such a testing unit.

The testing unit 502 is useful when testing one or more subsystems of the surgical robotic system. The testing unit can couple to the operator console to enable testing of the output generated by the operator console and/or the response of the operator console to emulated signals generated in dependence on the operator console output signals. An example of a way in which this testing can be advantageous is now provided, but it will be understood that this is merely exemplary of many different ways in which a testing unit such as the one described herein can be used.

The operator console is typically used to drive the motion of surgical robotic arms and instruments that form part of the bedside units described herein. It may be desired to troubleshoot or test a new drive algorithm, or to identify the occurrence of a fault in an existing drive algorithm. Use of the testing unit 502 can enable the operator console to be tested without needing the bulkier bedside units to be provided during the test. The bedside units can therefore be used, in conjunction with a different operator console, to perform a surgical procedure, increasing the operational efficiency of the system as a whole. Further, a single testing unit can be used together with an operator console in place of multiple bedside units. This reduces the space requirements for the test, not only for the dimensions of the bedside units themselves, but also for the space needed to ensure arm clearances as the robot arms and instruments move in response to control signals output by the operator console.

Use of a testing unit can be particularly effective where collision avoidance is to be tested. Using bedside units with the operator console runs the risk of arm collisions occurring, which can damage the arms, requiring maintenance and possible replacement. Where the testing unit is used with the operator console in place of the bedside units, it can be determined from the testing unit whether or not an arm collision would have occurred.

Thus, the use of the testing unit can increase the efficiency and help reduce potential costs of testing procedures.

The testing unit can be used to recover the system efficiently after a fault, e.g. a collision, has occurred, for example during a surgical procedure. Such fault recovery can for example be achieved by using the testing unit to emulate data such as telemetry data from a portion of the system that may have experienced the fault. This emulated data can then be analysed, for example compared with data from the portion of the system that may have experienced the fault. The comparison of the emulated data (which could be data expected where no fault has occurred) with data from the potentially faulty system portion can help reveal whether the fault has caused any damage to occur and/or whether it remains safe to continue using the system as a whole or that portion of the system in particular. The comparison can comprise comparing characteristic values of the data. The comparison can comprise comparing signal profiles.

The testing unit may be configured to emulate data in accordance with a fault having occurred in the system portion being emulated. In this case, the testing unit may generate multiple data streams. Each data stream may be generated in accordance with a different fault, or different set of fault conditions, being emulated. For example, a first fault condition to be emulated can comprise a first type of collision (for example a collision of an arm with a tabletop). A second fault condition to be emulated can comprise a second type of collision (for example a collision between two arms). A third fault condition to be emulated can comprise an arm actuator becoming faulty. It will be appreciated that many other faults and so on can similarly be emulated, including combinations: for example a fourth fault condition to be emulated can comprise the first type of collision and an arm actuator becoming faulty.

The emulated data streams can be compared with the data obtained from the system portion under test. The comparison can help identify the fault or set of fault conditions that has occurred. For instance, the emulated data stream that most closely matches the obtained data (e.g. in accordance with known comparison metrics, for example overall signal profile, comparing predefined characteristics of the signal, and so on) can be identified and the fault condition or set of fault conditions associated with that emulated data stream can be identified as the fault condition or set of fault conditions that has occurred, or is most likely to have occurred. As above, the comparison can comprise comparing characteristic values of the data streams. The comparison can comprise comparing signal profiles.

It may not always be possible to correctly identify the fault condition in this way. However, the identification of a most likely fault condition can help save time in later analysis and maintenance tasks, so the testing unit can still provide a benefit.

An example will now be discussed in the context of a collision of an arm with another object. The testing unit can be coupled to the arm in question. The testing unit may analyse data from the arm, for example by comparing telemetry data from the arm with an expected signal, such as one generated at the testing unit itself, or accessible to the testing unit, to see whether the collision of the arm caused any mechanical damage to the arm or whether it is safe to continue using the arm and/or the system as a whole. The comparison may be carried out for example by comparing the force of impact of the collision (as determined from the arm telemetry data) to a threshold value (generated at or accessible to the testing unit). The analysis may be carried out by running a test on the arm (such as by driving it using the testing unit) and analysing the signals received from the actuators to ensure they are working correctly.

This approach of using the testing unit can increase efficiency by removing the need to replace a bedside unit that is not damaged and is still working well and is safe to use.

The testing unit can be used for design verification and/or specification testing. The testing unit is suitably able to test subsystems such as the operator console and bedside units to ensure that those subsystems operate in line with the design requirements. For example, to ensure that a response to a given emulated signals is in line with the design specification for that subsystem. The use of the testing unit enables an accurate review of the responses of those subsystems to be made, without necessitating careful control by a human operator of, for example, the input controllers of the operator console, and/or manipulation of robotic arms.

Aspects of the testing unit will now be further described.

The subsystem to which the testing unit is configured to couple (which might be the operator console or a bedside unit) is, as discussed elsewhere, able to generate a first signal having a first characteristic behaviour. The boundary of the characteristic behaviour suitably comprises a maximum signal value. The testing unit is configured to generate the emulated signal with a value greater than the maximum signal value.

The boundary of the characteristic behaviour may comprise a minimum signal value. The testing unit is configured to generate the emulated signal with a value less than the minimum signal value. Suitably, the testing unit is configured to generate the emulated signal over a range of values that encompasses a first range of values of the first signal. For example, where the subsystem under test is able to generate a signal with a range of values of [x, y], the testing unit is suitably able to generate the emulated signal with a range of values of [p, q], where (i) p < x; and/or (ii) q > y.

The boundary of the first characteristic behaviour may comprise a repeatability indication of the first signal. The repeatability indication is an indication of how repeatable a signal value is. The repeatability indication may comprise a standard deviation. The testing unit is configured to generate the emulated signal with a repeatability greater than the indicated repeatability. That is, the emulated signal value can be more stable than the first signal value. The increased signal stability can assist in more accurate testing procedures.

The boundary of the first characteristic behaviour may comprise a rate of change of values of the first signal. The testing unit is suitably configured to generate the emulated signal with a rate of change greater than the boundary rate of change. This can enable the subsystem to be 'stress-tested' by applying a greater rate of change than would be applied during standard operation of the surgical robotic system. This can enable the testing unit to test that the subsystem under test is able to appropriately respond at faster processing speeds than should be required during such standard operation.

The testing unit is suitably configured to generate the emulated signal with a rate of change less than the boundary rate of change. Thus, the testing unit can check that the subsystem under test is able to respond appropriately to more slowly time-varying signals. This may be used to test the response to signal drift over time, for example.

The testing unit is suitably configured to generate one or more emulated signals from a set of emulated signals, each emulated signal in the set of emulated signals being representative of a signal that can be generated by the first subsystem. The first subsystem is suitably configured to be operational in one of a group of modes. For example, there may be an operative control mode, in which the operator console is operatively coupled to drive a surgical robot arm and operate a surgical robotic instrument. There may also be a clutch mode, in which the operator console is configured such that operator inputs do not drive at least one instrument. Many other modes are possible. The set of emulated signals can correspond to the group of modes of the surgical robotic system. For example, one emulated signal of the set of emulated signals can correspond to the operative control mode. Another emulated signal of the set of emulated signals can correspond to the clutch mode. Selection of an emulated signal from the set of emulated signals can therefore be used to emulate a selective enable and/or disable of various of the control modes possible in the surgical robotic system.

The testing unit need not just couple to one subsystem of the surgical robotic system. In some implementations, the testing unit can couple to more than one subsystem. The different subsystems to which the testing unit is configured to couple need not be of the same type. For example, the testing unit can couple both to an operator console and to a bedside unit. Suitably, the testing unit is configured to emulate the second subsystem by generating a further emulated signal representative of a second signal of the second subsystem for receiving at the first subsystem, the second signal having a second characteristic behaviour. The further emulated signal suitably has an emulated behaviour that exceeds a further boundary of the second characteristic behaviour of the second signal, such that the testing unit is operable to test the first subsystem beyond the capability of the second subsystem. Suitably, the testing unit is configured to emulate the second subsystem by transmitting the generated further emulated signal for receiving at the first subsystem. The testing unit can further be configured to emulate the second subsystem by receiving a further response signal from the first subsystem indicative of the response of the first subsystem to the further emulated signal. The testing unit is suitably configured to compare the received further response signal with a further expected signal, and to determine a state of the first subsystem based on the comparison.

The emulated signal can be representative of an arm control signal, for example a signal that commands movement of a robot arm. The emulated signal can comprise one or more desired joint torques and/or speeds, for example of one or more joints of the robot arm.

The emulated signal can be representative of an instrument control signal, for example a signal that commands movement of an instrument. The instrument is suitably coupled to a surgical robot arm, and the instrument control signal can be for transmission to the instrument via the robot arm. The emulated signal can comprise one or more desired joint torques and/or speeds, for example of one or more joints of the instrument. The emulated signal can comprise a desired instrument state, for example the opening angle between a pair of jaws of a grasper tool. The emulated signal may comprise an electrosurgical signal, such as the electrical activation level of an electrosurgical tool. The electrical activation level may comprise a voltage to be applied to the tool, or a power level for the tool.

The emulated signal may comprise a desired range of movement of the arm and/or of the instrument.

The testing unit described herein may be provided as part of a surgical system. The surgical system may comprise a surgical robotic system having the first subsystem (e.g. an operator console or a bedside unit) configured to generate a first signal having a first characteristic behaviour, and a second subsystem (e.g. a bedside unit or an operator console) configured to receive the first signal and to respond to the received first signal. The surgical system may further comprise the surgical robotic testing unit.

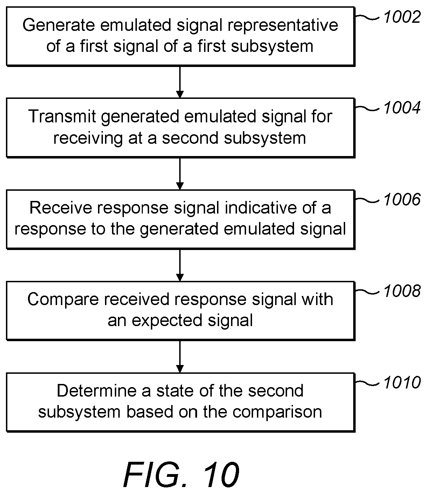

A method of operating the testing unit will now be described with reference to figure 10. The testing unit is operable to generate an emulated signal representative of the first signal of the first subsystem 1002. The emulated signal has an emulated behaviour that exceeds a boundary of the first characteristic behaviour of the first signal. The testing unit transmits the generated emulated signal for receiving at the second subsystem 1004.

The testing unit receives a response signal, from the second subsystem, indicative of the response of the second subsystem to the emulated signal 1006. The testing unit analyses the received response signal, for example by comparing the received response signal with an expected signal 1008, and determines a state of the second subsystem based on the analysis, for example the comparison 1010. Thus, the testing unit is operable to test the second subsystem beyond the capability of the first subsystem.

The testing unit can comprise a stand-alone module. The testing unit may therefore be provided with the necessary processors and so on, and transceivers for communicating with the subsystems. The testing unit can comprise multiple modules which may be physically or conceptually separate. For example, a processing module may be coupled to a data storage and transmission module. In some examples, the testing unit comprises a computing device such as a laptop or tablet computer. The functionality of the testing unit as described herein may be provided across any one or more, or combination of two or more, of such modules.

Any of the processors or kinematics controllers herein described may be microprocessors, controllers or any other suitable type of processor for processing computer executable instructions. In some examples, for example where a system-on-a-chip architecture is used, the processors and/or kinematics controllers may include one or more fixed function blocks (also referred to as accelerators) which implement a part of the method described herein in hardware (rather than software or firmware). Platform software comprising an operating system or any other suitable platform software may be provided to enable application software, such as software implementing the method of figure 10, to be executed.

The computer executable instructions may be provided using any computer-readable media that is accessible by a computing-based device. Computer-readable media may include, for example, computer storage media such as a memory and communications media. Computer storage media (i.e. non-transitory machine-readable media), such as the memory, includes volatile and non-volatile, removable and non-removable media implemented in any method or technology for storage of information such as computer readable instructions, data structures, program modules or other data. Computer storage media includes, but is not limited to, RAM, ROM, EPROM, EEPROM, flash memory or other memory technology, CD-ROM, digital versatile disks (DVD) or other optical storage, magnetic cassettes, magnetic tape, magnetic disk storage or other magnetic storage devices, or any other non-transmission medium that can be used to store information for access by a computing-based device. In contrast, communication media may embody computer readable instructions, data structures, program modules, or other data in a modulated data signal, such as a carrier wave, or other transport mechanism. As defined herein, computer storage media does not include communication media. Although the computer storage media (i.e. non-transitory machine-readable media, e.g. the memory) may be within the computing-based device, it will be appreciated that the storage may be distributed or located remotely and accessed via a network or other communication link (e.g. using a communication interface).

In the description above actions taken by the system have been split into functional blocks or modules for ease of explanation. In practice, two or more of these blocks could be architecturally combined. The functions could also be split into different functional blocks. The present techniques have been described in the context of surgical robotic systems, though at least some features described are not limited to such systems, but may be applied more generally to robotic systems comprising various subsystem that communicate with one another.

Robotic systems can include manufacturing systems, such as vehicle manufacturing systems, parts handling systems, laboratory systems, and manipulators such as for hazardous materials or surgical manipulators.

## Claims

1. A surgical robotic testing unit (502) for testing a surgical robotic system (500), the surgical robotic system (500) comprising a first subsystem (402, 404, 406, 408) configured to generate a first signal having a first characteristic behaviour and a second subsystem (402, 404, 406, 408) configured to receive the first signal and to respond to the received first signal, the testing unit comprising a memory configured to store a first signal relating to the first subsystem, and a database configured to store testing routines, wherein the testing unit (502) is configured to:
emulate the first subsystem (402, 404, 406, 408) by:
generating (1002) an emulated signal representative of the first signal of the first subsystem (402, 404, 406, 408) in accordance with a testing routine, the emulated signal having an emulated behaviour that exceeds a boundary of the first characteristic behaviour of the first signal, such that the testing unit (502) is operable to test the second subsystem (402, 404, 406, 408) beyond the capability of the first subsystem (402, 404, 406, 408);
transmitting (1004) the generated emulated signal for receiving at the second subsystem (402, 404, 406, 408); and
receiving (1006) a response signal from the second subsystem (402, 404, 406, 408) indicative of the response of the second subsystem (402, 404, 406, 408) to the emulated signal;
analysing the received response signal; and
determining (1010) a state of the second subsystem (402, 404, 406, 408) based on the analysis.

2. A surgical robotic testing unit (502) according to claim 1, in which the testing unit (502) is configured to analyse the received response signal by comparing the received response signal with an expected signal.

3. A surgical robotic testing unit (502) according to claim 1 or claim 2, in which:
the boundary of the first characteristic behaviour comprises a maximum signal value, and the testing unit (502) is configured to generate the emulated signal with a value greater than the maximum signal value; and/or
the boundary of the first characteristic behaviour comprises a minimum signal value, and the testing unit (502) is configured to generate the emulated signal with a value less than the minimum signal value.

4. A surgical robotic testing unit (502) according to any preceding claim, in which the testing unit (502) is configured to generate the emulated signal over a range of values that encompasses a first range of values of the first signal.

5. A surgical robotic testing unit (502) according to any preceding claim, in which the boundary of the first characteristic behaviour comprises a repeatability indication of the first signal, and the testing unit (502) is configured to generate the emulated signal with a repeatability greater than the indicated repeatability.

6. A surgical robotic testing unit (502) according to claim 5, in which the repeatability indication comprises a standard deviation, and the testing unit (502) is configured to generate the emulated signal with a standard deviation smaller than that of the repeatability indication.

7. A surgical robotic testing unit (502) according to any preceding claim, in which the boundary of the first characteristic behaviour comprises a rate of change of values of the first signal, and the testing unit (502) is configured to generate the emulated signal with a rate of change greater than and/or less than the boundary rate of change.

8. A surgical robotic testing unit (502) according to any preceding claim, in which the testing unit (502) is configured to generate one or more emulated signals from a set of emulated signals, each emulated signal in the set of emulated signals being representative of a signal that can be generated by the first subsystem (402, 404, 406, 408), and optionally in which the first subsystem (402, 404, 406, 408) is configured to be operational in one of a group of modes, and the set of emulated signals corresponds to the group of modes.

9. A surgical robotic testing unit (502) according to any preceding claim, in which the memory comprised by the testing unit (502) is configured to store a second signal relating to the second subsystem, and the testing unit is configured to:
emulate the second subsystem (402, 404, 406, 408) by:
generating a further emulated signal representative of the second signal of the second subsystem (402, 404, 406, 408) for receiving at the first subsystem (402, 404, 406, 408) in accordance with a testing routine, the second signal having a second characteristic behaviour, and the further emulated signal having an emulated behaviour that exceeds a further boundary of the second characteristic behaviour of the second signal, such that the testing unit (502) is operable to test the first subsystem (402, 404, 406, 408) beyond the capability of the second subsystem (402, 404, 406, 408);
transmitting the generated further emulated signal for receiving at the first subsystem (402, 404, 406, 408); and
receiving a further response signal from the first subsystem (402, 404, 406, 408) indicative of the response of the first subsystem (402, 404, 406, 408) to the further emulated signal;
analysing the received further response signal; and
determining a state of the first subsystem (402, 404, 406, 408) based on the analysis.

10. A surgical robotic testing unit (502) according to any preceding claim, in which the first subsystem (402, 404, 406, 408) comprises one of a control console (402) and a bedside unit (404, 406, 408) and the second subsystem (402, 404, 406, 408) comprises the other of the control console (402) and the bedside unit (404, 406, 408).

11. A surgical robotic testing unit (502) according to any preceding claim, in which the testing unit (502) is configured to emulate a plurality of control consoles (402) and/or bedside units (404, 406, 408).

12. A surgical robotic testing unit (502) according to any preceding claim, in which the emulated signal is representative of:
an arm control signal; and/or
an instrument control signal.

13. A surgical robotic testing unit (502) according to any preceding claim, in which the emulated signal comprises any one or more of:
one or more desired joint torques and/or speeds;
an electrosurgical signal; and
a desired range of movement of the arm and/or of the instrument.

14. A surgical system comprising:
a surgical robotic system (500) having
a first subsystem (402, 404, 406, 408) configured to generate a first signal having a first characteristic behaviour, and
a second subsystem (402, 404, 406, 408) configured to receive the first signal and to respond to the received first signal; and
comprising a surgical robotic testing unit (502) as claimed in claim 1.

15. A method of testing a surgical robotic system (500), the surgical robotic system (500) comprising a first subsystem (402, 404, 406, 408) configured to generate a first signal having a first characteristic behaviour, a second subsystem (402, 404, 406, 408) configured to receive the first signal and to respond to the received first signal, and a testing unit (502) comprising a memory configured to store the first signal and a database configured to store testing routines, the method comprising:
generating (1002) an emulated signal representative of the first signal of the first subsystem (402, 404, 406, 408) in accordance with a testing routine, the emulated signal having an emulated behaviour that exceeds a boundary of the first characteristic behaviour of the first signal;
transmitting (1004) the generated emulated signal for receiving at the second subsystem (402, 404, 406, 408);
receiving (1006) a response signal from the second subsystem (402, 404, 406, 408) indicative of the response of the second subsystem (402, 404, 406, 408) to the emulated signal;
analysing the received response signal; and
determining (1010) a state of the second subsystem (402, 404, 406, 408) based on the analysis, thereby testing the second subsystem (402, 404, 406, 408) beyond the capability of the first subsystem (402, 404, 406, 408).

## Patentansprüche

1. Chirurgische Robotertesteinheit (502) zum Testen eines chirurgischen Robotersystems (500), wobei das chirurgische Robotersystem (500) ein erstes Subsystem (402, 404, 406, 408), das konfiguriert ist, ein erstes Signal mit einem ersten charakteristischen Verhalten zu erzeugen, und ein zweites Subsystem (402, 404, 406, 408), das konfiguriert ist, das erste Signal zu empfangen und auf das empfangene erste Signal zu reagieren, umfasst, wobei die Testeinheit einen Speicher, der konfiguriert ist, ein erstes Signal im Zusammenhang mit dem ersten Subsystem zu speichern, und eine Datenbank, die konfiguriert ist, Testroutinen zu speichern, umfasst, wobei die Testeinheit (502) konfiguriert ist zum:
Emulieren des ersten Subsystems (402, 404, 406, 408) durch:
Erzeugen (1002) eines emulierten Signals, das das erste Signal des ersten Subsystems (402, 404, 406, 408) gemäß einer Testroutine darstellt, wobei das emulierte Signal ein emuliertes Verhalten aufweist, das eine Grenze des ersten charakteristischen Verhaltens des ersten Signals übersteigt, sodass die Testeinheit (502) betreibbar ist, um das zweite Subsystem (402, 404, 406, 408) über die Fähigkeit des ersten Subsystems (402, 404, 406, 408) hinaus zu testen;
Senden (1004) des erzeugten emulierten Signals zum Empfangen an dem zweiten Subsystem (402, 404, 406, 408); und
Empfangen (1006) eines Antwortsignals von dem zweiten Subsystem (402, 404, 406, 408), das die Antwort des zweiten Subsystems (402, 404, 406, 408) auf das emulierte Signal angibt;
Analysieren des empfangenen Antwortsignals; und
Bestimmen (1010) eines Zustands des zweiten Subsystems (402, 404, 406, 408) basierend auf der Analyse.

2. Chirurgische Robotertesteinheit (502) nach Anspruch 1, wobei die Testeinheit (502) konfiguriert ist, das empfangene Antwortsignal durch Vergleichen des empfangenen Antwortsignals mit einem erwarteten Signal zu analysieren.

3. Chirurgische Robotertesteinheit (502) nach Anspruch 1 oder Anspruch 2, wobei:
die Grenze des ersten charakteristischen Verhaltens einen maximalen Signalwert umfasst, und die Testeinheit (502) konfiguriert ist, das emulierte Signal mit einem Wert zu erzeugen, der größer als der maximale Signalwert ist; und/oder
die Grenze des ersten charakteristischen Verhaltens einen minimalen Signalwert umfasst und die Testeinheit (502) konfiguriert ist, das emulierte Signal mit einem Wert zu erzeugen, der kleiner als der minimale Signalwert ist.

4. Chirurgische Robotertesteinheit (502) nach einem der vorhergehenden Ansprüche, wobei die Testeinheit (502) konfiguriert ist, das emulierte Signal über einen Bereich von Werten zu erzeugen, der einen ersten Bereich von Werten des ersten Signals umfasst.

5. Chirurgische Robotertesteinheit (502) nach einem der vorhergehenden Ansprüche, wobei die Grenze des ersten charakteristischen Verhaltens eine Wiederholbarkeitsangabe des ersten Signals umfasst und die Testeinheit (502) konfiguriert ist, das emulierte Signal mit einer Wiederholbarkeit zu erzeugen, die größer als die angezeigte Wiederholbarkeit ist.

6. Chirurgische Robotertesteinheit (502) nach Anspruch 5, wobei die Wiederholbarkeitsangabe eine Standardabweichung umfasst und die Testeinheit (502) konfiguriert ist, das emulierte Signal mit einer Standardabweichung zu erzeugen, die kleiner als die der Wiederholbarkeitsangabe ist.

7. Chirurgische Robotertesteinheit (502) nach einem der vorhergehenden Ansprüche, wobei die Grenze des ersten charakteristischen Verhaltens eine Änderungsrate von Werten des ersten Signals umfasst und die Testeinheit (502) konfiguriert ist, das emulierte Signal mit einer Änderungsrate zu erzeugen, die größer als und/oder kleiner als die Grenzänderungsrate ist.

8. Chirurgische Robotertesteinheit (502) nach einem der vorhergehenden Ansprüche, wobei die Testeinheit (502) konfiguriert ist, ein oder mehrere emulierte Signale aus einem Satz emulierter Signale zu erzeugen, wobei jedes emulierte Signal in dem Satz emulierter Signale repräsentativ für ein Signal ist, das durch das erste Subsystem (402, 404, 406, 408) erzeugt werden kann, und wobei optional das erste Subsystem (402, 404, 406, 408) konfiguriert ist, in einem von einer Gruppe von Modi betrieben zu werden, und der Satz emulierter Signale der Gruppe von Modi entspricht.

9. Chirurgische Robotertesteinheit (502) nach einem der vorhergehenden Ansprüche, wobei der von der Testeinheit (502) umfasste Speicher konfiguriert ist, ein zweites Signal zu speichern, das sich auf das zweite Subsystem bezieht, und die Testeinheit konfiguriert ist zum:
Emulieren des zweiten Subsystems (402, 404, 406, 408) durch:
Erzeugen eines weiteren emulierten Signals, das für das zweite Signal des zweiten Subsystems (402, 404, 406, 408) repräsentativ ist, zum Empfangen an dem ersten Subsystem (402, 404, 406, 408) gemäß einer Testroutine, wobei das zweite Signal ein zweites charakteristisches Verhalten aufweist und das weitere emulierte Signal ein emuliertes Verhalten aufweist, das eine weitere Grenze des zweiten charakteristischen Verhaltens des zweiten Signals übersteigt, sodass die Testeinheit (502) betreibbar ist, um das erste Subsystem (402, 404, 406, 408) über die Fähigkeit des zweiten Subsystems (402, 404, 406, 408) hinaus zu testen;
Senden des erzeugten weiteren emulierten Signals zum Empfangen an dem ersten Subsystem (402, 404, 406, 408); und
Empfangen eines weiteren Antwortsignals von dem ersten Subsystem (402, 404, 406, 408), das die Antwort des ersten Subsystems (402, 404, 406, 408) auf das weitere emulierte Signal angibt;
Analysieren des empfangenen weiteren Antwortsignals; und
Bestimmen eines Zustands des ersten Subsystems (402, 404, 406, 408) basierend auf der Analyse.

10. Chirurgische Robotertesteinheit (502) nach einem der vorhergehenden Ansprüche, wobei das erste Subsystem (402, 404, 406, 408) entweder eine Steuerkonsole (402) oder eine Bettseiteneinheit (404, 406, 408) umfasst und das zweite Subsystem (402, 404, 406, 408) das andere von der Steuerkonsole (402) und der Bettseiteneinheit (404, 406, 408) umfasst.

11. Chirurgische Robotertesteinheit (502) nach einem der vorhergehenden Ansprüche, wobei die Testeinheit (502) konfiguriert ist, eine Mehrzahl von Steuerkonsolen (402) und/oder Bettseiteneinheiten (404, 406, 408) zu emulieren.

12. Chirurgische Robotertesteinheit (502) nach einem der vorhergehenden Ansprüche, wobei das emulierte Signal repräsentativ ist für:
ein Armsteuersignal; und/oder
ein Instrumentensteuersignal.

13. Chirurgische Robotertesteinheit (502) nach einem der vorhergehenden Ansprüche, wobei das emulierte Signal eines oder mehrere der folgenden umfasst:
ein oder mehrere gewünschte Gelenkdrehmomente und/oder -geschwindigkeiten;
ein elektrochirurgisches Signal; und
einen gewünschten Bewegungsbereich des Arms und/oder des Instruments.

14. Chirurgisches System, umfassend:
ein chirurgisches Robotersystem (500), aufweisend
ein erstes Subsystem (402, 404, 406, 408), das so konfiguriert ist, dass es ein erstes Signal mit einem ersten charakteristischen Verhalten erzeugt, und
ein zweites Subsystem (402, 404, 406, 408), das konfiguriert ist, das erste Signal zu empfangen und auf das empfangene erste Signal zu reagieren; und
**dadurch gekennzeichnet, dass** es eine chirurgische Robotertesteinheit (502) nach Anspruch 1 umfasst.

15. Verfahren zum Testen eines chirurgischen Robotersystems (500), wobei das chirurgische Robotersystem (500) ein erstes Subsystem (402, 404, 406, 408), das konfiguriert ist, ein erstes Signal mit einem ersten charakteristischen Verhalten zu erzeugen, ein zweites Subsystem (402, 404, 406, 408), das konfiguriert ist, das erste Signal zu empfangen und auf das empfangene erste Signal zu reagieren, und eine Testeinheit (502), die einen Speicher aufweist, der konfiguriert ist, das erste Signal zu speichern, und eine Datenbank, die konfiguriert ist, Testroutinen zu speichern, umfasst, das Verfahren umfassend:
Erzeugen (1002) eines emulierten Signals, das das erste Signal des ersten Subsystems (402, 404, 406, 408) darstellt, gemäß einer Testroutine, wobei das emulierte Signal ein emuliertes Verhalten aufweist, das eine Grenze des ersten charakteristischen Verhaltens des ersten Signals überschreitet;
Senden (1004) des erzeugten emulierten Signals zum Empfangen durch das zweite Subsystem (402, 404, 406, 408);
Empfangen (1006) eines Antwortsignals von dem zweiten Subsystem (402, 404, 406, 408), das die Antwort des zweiten Subsystems (402, 404, 406, 408) auf das emulierte Signal angibt;
Analysieren des empfangenen Antwortsignals; und
Bestimmen (1010) eines Zustands des zweiten Subsystems (402, 404, 406, 408) auf Grundlage der Analyse, wodurch das zweite Subsystem (402, 404, 406, 408) über die Fähigkeiten des ersten Subsystems (402, 404, 406, 408) hinaus getestet wird.

## Revendications

1. Unité de test robotique chirurgicale (502) permettant de tester un système robotique chirurgical (500), le système robotique chirurgical (500) comprenant un premier sous-système (402, 404, 406, 408) configuré pour générer un premier signal ayant un premier comportement caractéristique et un deuxième sous-système (402, 404, 406, 408) configuré pour recevoir le premier signal et pour répondre au premier signal reçu, l'unité de test comprenant une mémoire configurée pour stocker un premier signal relatif au premier sous-système, et une base de données configurée pour stocker des routines de test, dans laquelle l'unité de test (502) est configurée pour :
émuler le premier sous-système (402, 404, 406, 408) par :
la génération (1002) d'un signal émulé représentatif du premier signal du premier sous-système (402, 404, 406, 408) conformément à une routine de test, le signal émulé ayant un comportement émulé qui dépasse une limite du premier comportement caractéristique du premier signal, de telle sorte que l'unité de test (502) peut fonctionner pour tester le deuxième sous-système (402, 404, 406, 408) au-delà de la capacité du premier sous-système (402, 404, 406, 408) ;
la transmission (1004) du signal émulé généré pour la réception au niveau du deuxième sous-système (402, 404, 406, 408) ; et
la réception (1006) d'un signal de réponse en provenance du deuxième sous-système (402, 404, 406, 408) indicatif de la réponse du deuxième sous-système (402, 404, 406, 408) au signal émulé ;
l'analyse du signal de réponse reçu ; et
la détermination (1010) d'un état du deuxième sous-système (402, 404, 406, 408) sur la base de l'analyse.

2. Unité de test robotique chirurgicale (502) selon la revendication 1, dans laquelle l'unité de test (502) est configurée pour analyser le signal de réponse reçu en comparant le signal de réponse reçu à un signal attendu.

3. Unité de test robotique chirurgicale (502) selon la revendication 1 ou la revendication 2, dans laquelle :
la limite du premier comportement caractéristique comprend une valeur de signal maximale, et l'unité de test (502) est configurée pour générer le signal émulé avec une valeur supérieure à la valeur de signal maximale ; et/ou
la limite du premier comportement caractéristique comprend une valeur de signal minimale, et l'unité de test (502) est configurée pour générer le signal émulé avec une valeur inférieure à la valeur de signal minimale.

4. Unité de test robotique chirurgicale (502) selon une quelconque revendication précédente, dans laquelle l'unité de test (502) est configurée pour générer le signal émulé sur une plage de valeurs qui englobe une première plage de valeurs du premier signal.

5. Unité de test robotique chirurgicale (502) selon une quelconque revendication précédente, dans laquelle la limite du premier comportement caractéristique comprend une indication de répétabilité du premier signal, et l'unité de test (502) est configurée pour générer le signal émulé avec une répétabilité supérieure à la répétabilité indiquée.

6. Unité de test robotique chirurgicale (502) selon la revendication 5, dans laquelle l'indication de répétabilité comprend un écart-type, et l'unité de test (502) est configurée pour générer le signal émulé avec un écart-type inférieur à celui de l'indication de répétabilité.

7. Unité de test robotique chirurgicale (502) selon une quelconque revendication précédente, dans laquelle la limite du premier comportement caractéristique comprend un taux de variation de valeurs du premier signal, et l'unité de test (502) est configurée pour générer le signal émulé avec un taux de variation supérieur et/ou inférieur au taux de variation limite.

8. Unité de test robotique chirurgicale (502) selon une quelconque revendication précédente, dans laquelle l'unité de test (502) est configurée pour générer un ou plusieurs signaux émulés à partir d'un ensemble de signaux émulés, chaque signal émulé dans l'ensemble de signaux émulés étant représentatif d'un signal qui peut être généré par le premier sous-système (402, 404, 406, 408), et facultativement dans laquelle le premier sous-système (402, 404, 406, 408) est configuré pour être fonctionnel dans l'un parmi un groupe de modes, et l'ensemble de signaux émulés correspond au groupe de modes.

9. Unité de test robotique chirurgicale (502) selon une quelconque revendication précédente, dans laquelle la mémoire comprise par l'unité de test (502) est configurée pour stocker un deuxième signal concernant le deuxième sous-système, et l'unité de test est configurée pour :
émuler le deuxième sous-système (402, 404, 406, 408) par :
la génération d'un signal émulé supplémentaire représentatif du deuxième signal du deuxième sous-système (402, 404, 406, 408) pour recevoir au niveau du premier sous-système (402, 404, 406, 408) conformément à une routine de test, le deuxième signal ayant un deuxième comportement caractéristique, et le signal émulé supplémentaire ayant un comportement émulé qui dépasse une limite supplémentaire du deuxième comportement caractéristique du deuxième signal, de telle sorte que l'unité de test (502) est utilisable pour tester le premier sous-système (402, 404, 406, 408) au-delà de la capacité du deuxième sous-système (402, 404, 406, 408) ;
la transmission du signal émulé supplémentaire généré pour la réception au niveau du premier sous-système (402, 404, 406, 408) ; et
la réception d'un signal de réponse supplémentaire en provenance du premier sous-système (402, 404, 406, 408) indicatif de la réponse du premier sous-système (402, 404, 406, 408) au signal émulé supplémentaire ;
l'analyse du signal de réponse supplémentaire reçu ; et
la détermination d'un état du premier sous-système (402, 404, 406, 408) sur la base de l'analyse.

10. Unité de test robotique chirurgicale (502) selon une quelconque revendication précédente, dans laquelle le premier sous-système (402, 404, 406, 408) comprend l'une parmi une console de commande (402) et une unité de chevet (404, 406, 408), et le deuxième sous-système (402, 404, 406, 408) comprend l'autre parmi la console de commande (402) et l'unité de chevet (404, 406, 408).

11. Unité de test robotique chirurgicale (502) selon une quelconque revendication précédente, dans laquelle l'unité de test (502) est configurée pour émuler une pluralité de consoles de commande (402) et/ou d'unités de chevet (404, 406, 408).

12. Unité de test robotique chirurgicale (502) selon une quelconque revendication précédente, dans laquelle le signal émulé est représentatif :
d'un signal de commande de bras ; et/ou
d'un signal de commande d'instrument.

13. Unité de test robotique chirurgicale (502) selon une quelconque revendication précédente, dans laquelle le signal émulé comprend un ou plusieurs parmi :
un(e) ou plusieurs couples et/ou vitesses d'articulation souhaités ;
un signal électrochirurgical ; et
une plage de mouvements souhaitée du bras et/ou de l'instrument.

14. Système chirurgical comprenant :
un système robotique chirurgical (500) ayant
un premier sous-système (402, 404, 406, 408) configuré pour générer un premier signal ayant un premier comportement caractéristique, et
un deuxième sous-système (402, 404, 406, 408) configuré pour recevoir le premier signal et pour répondre au premier signal reçu ; et
**caractérisé en ce qu'**il comprend une unité de test robotique chirurgicale (502) selon la revendication 1.

15. Procédé de test d'un système robotique chirurgical (500), le système robotique chirurgical (500) comprenant un premier sous-système (402, 404, 406, 408) configuré pour générer un premier signal ayant un premier comportement caractéristique, un deuxième sous-système (402, 404, 406, 408) configuré pour recevoir le premier signal et pour répondre au premier signal reçu, et une unité de test (502) comprenant une mémoire configurée pour stocker le premier signal et une base de données configurée pour stocker des routines de test, le procédé comprenant :
la génération (1002) d'un signal émulé représentatif du premier signal du premier sous-système (402, 404, 406, 408) conformément à une routine de test, le signal émulé ayant un comportement émulé qui dépasse une limite du premier comportement caractéristique du premier signal ;
la transmission (1004) du signal émulé généré pour la réception au niveau du deuxième sous-système (402, 404, 406, 408) ;
la réception (1006) d'un signal de réponse en provenance du deuxième sous-système (402, 404, 406, 408) indicatif de la réponse du deuxième sous-système (402, 404, 406, 408) au signal émulé ;
l'analyse du signal de réponse reçu ; et
la détermination (1010) d'un état du deuxième sous-système (402, 404, 406, 408) sur la base de l'analyse, et ainsi tester le deuxième sous-système (402, 404, 406, 408) au-delà de la capacité du premier sous-système (402, 404, 406, 408).
